# EUROPEAN PATENT APPLICATION

(11) **EP 4 714 378 A1**
(43) Date of publication of application: **25.03.2026**
(21) Application number: 24382994.2
(22) Date of filing: 18.09.2024
(51) Int. Cl.: A61B 18/08

(54) **MEDICAL COAGULATION INSTRUMENT, METHOD FOR MANUFACTURING A MEDICAL COAGULATION INSTRUMENT AND MEDICAL COAGULATION KIT**

(71) Applicant: Aesculap AG, 78532 Tuttlingen (DE)
(72) Inventor: TURÓN, Pau, 08191 RUBÍ (ES); SANZ, Vanesa, 08191 RUBÍ (ES); WEIS, Christine, 08190 Sant Cugat del Vallés (ES); VOGT, Lena, 78315 Radolfzell (DE); HUBER, Christian, 78570 Mühlheim (DE)
(74) Representative: Patentanwälte Ruff, Wilhelm, Beier, Dauster & Partner mbB

(57) **Abstract**

The invention relates to a medical coagulation instrument comprising an instrument body and thermal nanoparticles, wherein the thermal nanoparticles support optical resonance, in particular localized surface plasmon resonance, and are bonded to and/or entrapped in a surface of the instrument body, wherein the thermal nanoparticles are capable of increasing their temperature by light irradiation in a wavelength range that matches with the wavelength of the optical resonance, in particular localized surface plasmon resonance, of the thermal nanoparticles.

Further, the invention relates to a method for manufacturing a medical coagulation instrument, to a medical coagulation kit and a method for coagulation of blood.

## Description

### FIELD OF THE INVENTION

The present invention relates to a medical coagulation instrument, method for manufacturing a medical coagulation instrument, a medical coagulation kit and a method for coagulation of blood.

### BACKGROUND OF THE INVENTION

During surgeries, a coagulation of blood is often required to prevent excessive bleeding during surgery and ensure the success of the surgery (e.g., reduced risk of complications, support of healing). The increase in laparoscopic surgery, neurosurgery and general surgery has resulted in an increased need for safe and reliable methods of obtaining minimally invasive operative hemostasis. Therefore, basically monopolar, bipolar and ultrasound energy are used. More recently, laser irradiation is applied to induce blood coagulation in different surgical fields.

For example, bipolar coagulation is a proven technique for achieving occlusion of blood vessels and tissue bundles by application of high frequency electrical current. The tissue is held between two separate jaws of forceps and current is applied to the jaws that creates a thermal effect in the tissue due to passage of high frequency current and achieves occlusion which stops bleeding during surgical procedure. This technique is suitable for coagulation of tissue in approximately 3 mm up to 7 mm in size. Vessel sealing is another technique of applying bipolar current with specific pressure on the tissue.

A disadvantage using electrosurgical procedures for sealing of blood vessels with bipolar coagulation instruments is that the generated thermal energy spreads to the surrounding tissue and may irreversibly damage it. This is particularly problematic when operating close to sensitive structures such as nerves. A further disadvantage is that these instruments must be connected with a cable to a generator such as a HF (high frequency) generator. The cable influences the degrees of freedom in the application and can interfere with the surgical procedure. In addition, the instruments must be fitted with elaborate electrical insulation so that patients, users and third parties do not suffer any unwanted burns.

### OBJECT AND SOLUTION

In view of the foregoing, the object underlying the present invention is therefore to make available an optimized medical instrument which at least partly avoids the disadvantages occurring in the context of known medical instruments being applied for prevention and/or stop of bleeding.

This object is accomplished by a medical coagulation instrument according to independent claim 1, a method for manufacturing a medical coagulation instrument according to claim 14, a medical coagulation kit according to claim 15 and a method for coagulation of blood as disclosed in the description. Preferred embodiments of the invention are defined in the dependent claims and the present description. The subject-matter and wording, respectively, of all claims is hereby incorporated into the description by explicit reference.

According to a first aspect, the invention relates to a medical, in particular surgical, coagulation instrument, i.e., a medical, in particular surgical, instrument being adapted or configured to coagulate blood, i.e. to prevent and/or stop bleeding. In other words, the medical, in particular surgical, coagulation instrument according to the present invention refers to a medical, in particular surgical, instrument being adapted or configured to achieve hemostasis. Thus, the medical, in particular surgical, coagulation instrument according to the present invention may also be termed as a medical, in particular surgical, hemostasis instrument.

The medical coagulation instrument comprises an instrument body and thermal nanoparticles. The thermal nanoparticles support optical resonance, in particular localized surface plasmon resonance (LSPR), and are bonded to a surface of the instrument body and/or are entrapped in a surface of the instrument body. The thermal nanoparticles are capable of increasing their temperature by light irradiation in a wavelength range that matches with the wavelength of the optical resonance, in particular localized surface plasmon resonance, of the thermal nanoparticles.

Preferably, the thermal nanoparticles form a coating, in particular thermal coating, on the surface of the instrument body, wherein the coating, in particular thermal coating, rises its temperature upon light irradiation of the thermal nanoparticles at their optical wavelength, in particular localized surface plasmon resonance.

The coherent oscillation of electrons of the thermal nanoparticles driven by light irradiation causes an increase of the nanoparticles' temperature, whereby the thermal nanoparticles are additionally heated by thermal diffusion.

The term "nanoparticles" as used according to the present invention refers to particles having a mean particle diameter from 10 nm to 1 µm, in particular 10 nm to ≤ 100 nm, preferably 10 nm to < 100 nm. The mean particle diameter may be determined by means of conventional methods such as dynamic light scattering, transmission electron microscopy (TEM) or scanning electron microscopy (SEM).

The term "surface plasmon resonance" (SPR) as used according to the present invention refers to the collective resonant oscillation of electrons of a material excited by incident light (light irradiation). The resonance condition is established when the frequency (wavelength range) of light matches or couples the natural frequency (wavelength band) of quasi-free electrons oscillating against the restoring force of positive nuclei. SPR in nanometer-sized nanoparticles is also called localized surface plasmon resonance (LSPR).

The term "light irradiation" or "light irradiated" as used according to the present invention refers to the range of optical wavelengths used to rise the temperature of the thermal nanoparticles. The wavelength of this light should overlap with the wavelength optical resonance, in particular wavelength plasmon resonance, of the thermal nanoparticles. Preferably, the irradiated wavelength range is within 750 nm to 1200 nm, in particular 780 nm to 900 nm, preferably 800 nm to 840 nm, more preferably 800 nm to 820 nm, in particular 805 nm to 815 nm, preferably 810 nm. Especially, a wavelength of 805 nm to 815 nm, preferably 810 nm, advantageously increases penetration depth into tissue versus visible light with less penetration of surrounding tissue.

The term "thermal nanoparticles" as used according to the present invention refers to nanoparticles, in particular plasmonic nanoparticles, engineered to generate an electric field inside of the nanoparticles upon light irradiation. Thus, the generated electric field inside of the thermal nanoparticles is responsible for the heat generation of the thermal nanoparticles, and the power of heat generation inside the thermal nanoparticles is directly proportional to the absorption cross-section.

The thermal nanoparticles may be in particular plasmonic nanoparticles engineered to absorb into the nanoparticles the incident light upon light irradiation and mainly dissipate into the ions network the light intercepted by the nanoparticles and the corresponding energy stored in the electron cloud, generating a heating on the nanoparticles.

The term "entrapped in a surface of the instrument body" as used according to the present invention in terms of the thermal nanoparticles means that the thermal nanoparticles are dispersed or distributed, in particularly homogeneously or not homogeneously (randomly), in a material, in particular polymer material or ceramic material, that forms the surface of the instrument body, preferably wherein the thermal nanoparticles are dispersed or distributed on the surface of the instrument body or a part of the instrument body and/or the thermal nanoparticles are, in particular even completely, dispersed or distributed within the instrument body or a/the part of the instrument body.

The term "entrapping the selected thermal nanoparticles during manufacture of an instrument body or a part of an instrument body in a surface of the instrument body or part of the instrument body" as used according to the present invention means a step of dispersing or distributing, in particularly homogeneously or not homogeneously (randomly), of the thermal nanoparticles in a material, in particular polymer material or ceramic material, that forms the surface of the instrument body or part of the instrument body, preferably wherein the thermal nanoparticles are dispersed or distributed on the surface of the instrument body or part of the instrument body and/or the thermal nanoparticles are, in particular even completely, dispersed or distributed within the instrument body or a/the part of the instrument body.

The present invention is based on the surprising finding that bonding or entrapping of thermal nanoparticles supporting optical resonance, in particular localized surface plasmon resonance, to or in a surface of a medical instrument allows for blood coagulation. Thus, light energy can be transformed to heat to be precisely locally effective to coagulate blood and/or to seal blood vessels, in particular arteries and/or veins, in vivo. The increase of temperature, in particular hyperthermia, by irradiation can be performed easily and repeatedly as many times as necessary, since the thermal nanoparticles are fixed, preferably firmly, i.e. unreleasably, fixed on the surface of the medical instrument and/or are entrapped in the surface of the medical instrument. Advantageously, the temperature increase is immediate which increases the speed of blood coagulation with high accuracy, which results in less blood loss and less tissue damage and less adhesions at a necrosectomy site. More specifically, the thermal spread into tissue is low due to the rapidly raised temperature as well as the rapid cooling down when irradiation is stopped.

In an embodiment of the invention, the surface of the instrument body to which the thermal nanoparticles are bonded to and/or in which the thermal nanoparticles are entrapped comprises a surface of a least one distal tip portion, in particular of only one distal tip portion or of two or more, preferably of only two, distal tip portions, of the instrument body. The at least one distal tip portion may be in particular in the form of at least one distal jaw portion, in particular only one distal jaw portion or two or more, preferably only two, distal jaw portions.

The term "distal tip portion" as used according to the present invention refers to a tip portion of the instrument body facing away from the trunk of a user, in particular physician or medical staff, if the instrument body or medical coagulation instrument is used as intended.

The term "distal jaw portion" as used according to the present invention refers to a jaw portion of the instrument body facing away from the trunk of a user, in particular physician or medical staff, if the instrument body or medical coagulation instrument is used as intended.

In a further embodiment of the invention, the instrument body comprises two distal tip portions, wherein the surface to which the thermal nanoparticles are bonded to and/or in which the thermal nanoparticles are entrapped comprises a surface of at least one of the two distal tip portions, in particular a surface of only one of the two distal tip portions or a surface of both distal tip portions.

Preferably, the instrument body comprises two distal tip portions, wherein the surface to which the thermal nanoparticles are bonded to and/or in which the thermal nanoparticles are entrapped comprises at least one surface of two opposing surfaces of the two distal tip portions, in particular only one surface of two opposing surfaces of the two distal tip portions or both opposing surfaces of the two distal tip portions.

In a further embodiment of the invention, one of the two distal tip portions is part of a first instrument branch of the instrument body and the other distal tip portion is part of a second instrument branch of the instrument body.

In a further embodiment of the invention, the first instrument branch and second instrument branch are pivotally coupled to each other. Preferably, each of the first instrument branch and second instrument branch has a proximal handling portion and a distal tip portion, the distal tip portions forming an instrument mouth which is movable by a pivotal movement of the first instrument branch and second instrument branch between a closed state, in which the distal tip portions are moved inwardly towards each other, and an open state, in which the distal tip portions are moved outwardly away from each other.

In a further embodiment of the invention, the first instrument branch and second instrument branch are non-pivotally coupled to each other. Preferably, the first instrument branch and second instrument branch are connected with each other by a common juncture.

In a further embodiment of the invention, the instrument body comprises a base, i.e. main, portion, to which the at least one distal tip portion, in particular the two distal tip portions are connected, in particular positively connected and/or force-fit connected and/or materially connected.

In a further embodiment of the invention, the medical coagulation instrument is in the form of a single use medical coagulation instrument.

The term "single use medical coagulation instrument" as used according to the present invention refers to a medical coagulation instrument that is adapted or configured to be used once and then discarded.

In a further embodiment of the invention, a portion, in particular only a portion, of the medical coagulation instrument is in the form of a single use portion. The portion which may be in the form of a single use portion may be, for example, the base or main portion and/or at least one distal tip portion, in particular two distal tip portions, and/or proximal handling portion and/or common juncture of the medical coagulation instrument.

The term "single use portion" as used according to the present invention refers to a portion of the medical coagulation instrument which is configured to be used once and then discarded.

Preferably, the at least one distal tip portion, in particular two distal tip portions, of the medical coagulation instrument is in the form a single use distal tip portion, in particular two single use distal tip portions.

The term "single use distal tip portion" as used according to the present invention refers to a distal tip portion which is configured to be used once and then discarded.

In a further embodiment of the invention, the medical coagulation instrument is in the form of a surgical coagulation instrument, in particular selected from the group consisting of surgical grasping instrument, surgical clamping instrument and surgical sealing instrument, in particular vessel sealing instrument.

In a further embodiment of the invention, the medical, in particular surgical, coagulation instrument is selected from the group consisting of surgical tweezers, surgical scissors, surgical pliers, hemostatic clamps, arterial forceps, surgical staplers and scalpels.

In a further embodiment of the invention, the surface of the instrument body to which the thermal nanoparticles are bonded to and/or in which the thermal nanoparticles are entrapped is a metallic surface, i.e. a surface comprising or consisting of a metal or alloy. More specifically, the metallic surface may comprise or consist of a metal or alloy selected from the group consisting of steels, stainless steels, titanium, hard metals and combinations of at least two of the afore-mentioned metals or alloys. Alternatively or in combination, the metallic surface may comprise or consist of a metal or alloy as mentioned in DIN EN ISO 7153-1:2017-02.

In a further embodiment of the invention, the surface of the instrument body to which the thermal nanoparticles are bonded to and/or in which the thermal nanoparticles are entrapped is a polymeric surface, i.e. a surface comprising or consisting of a polymer. More specifically, the polymeric surface may comprise or consist of a polymer selected from the group consisting of polyolefin, polyethylene, low-density polyethylene, high-density polyethylene, high-molecular-weight polyethylene, ultra-high-molecular-weight polyethylene, polypropylene, polytetrafluorethylene, polyvinylidene fluoride, polyvinylidene chloride, polytetrafluorpropylene, polyhexafluorpropylene, polyester, polyethylene terephthalate, polypropylene terephthalate, polybutylene terephthalate, polyamide, polyamide 6 (polymer of ε-caprolactam- or omega-aminocaproic acid units), polyamide 66 (polymer of hexamethylenediamine units and adipic acid units), polyamide 69 (polymer of hexamethylenediamine units and azelaic acid units), polyamide 612 (polymer of hexamethylenediamine units and dodecanedioic acid units), polyamide 11 (polymer of 11-aminoundecanoic acid units), polyamide 12 (polymer of laurolactam acid units and omega-aminododecanoic acid units), polyamide 46 (polymer of tetramethylenediamine units and adipic acid units), polyamide 1212 (polymer of dodecandiamine units and dodecanedioic acid units), polyamide 6/12 (polymer of caprolactam units and laurolactam units), polyamide 66/610 (polymer of hexamethylenediamine units, adipic acid units and sebacic acid units), polyurethane, aliphatic polycarbonate urethane, silicon polycarbonate urethane, polyether urethane, silicone polyether urethane, polyurethane ether, polyetherketone, polyetherketoneketone, polyetheretheretherketone, polyetheretherketoneketone, polyetherketoneetherketoneketone, copolymers of at least two of the afore-mentioned polymers and combinations of at least two of the afore-mentioned polymers. With respect to further useful polymers, reference is made to the following description.

In a further embodiment of the invention, the surface of the instrument body to which the thermal nanoparticles are bonded to and/or in which the thermal nanoparticles are entrapped is a transparent surface. A transparent surface may advantageously additional foster the influence of light to the surface of the instrument body to be irradiated.

The term "transparent surface" as used according to the present invention refers to a surface which allows light, in particular having a wavelength of 650 nm to 1200 nm, in particular 650 nm to 1100 nm or 750 nm to 1200 nm, to pass through the surface, in particular without appreciable scattering of the light.

In particular, the transparent surface comprises or consists of a transparent polymer.

The term "transparent polymer" as used according to the present invention refers to a polymer which allows light, in particular having a wavelength of 650 nm to 1200 nm, in particular 650 nm to 1100 nm or 750 nm to 1200 nm, to pass through the polymer, in particular without appreciable scattering of the light.

Preferably, the transparent polymer is selected from the group consisting of polyethylene, polypropylene, polyimide, polyethylene terephthalate, silicone, polydimethylsiloxane, poly(methyl methacrylate), poly(phthalazinone ether sulfone ketone), poly(m-phenylene isophthalamide), polybenzoxazole, polycarbonate, cyclic olefin copolymer and combinations of at least two of the afore-mentioned transparent polymers. Preferably, the cyclic olefin copolymer is produced by chain copolymerization of cyclic monomers such as 8,9,10-trinorborn-2-ene (norbornene) or 1,2,3,4,4a,5,8,8a-octahydro-1,4:5,8-dimethanonaphthalene (tetracyclododecene) with ethene. More preferably, the cyclic olefin copolymer is ethylene-norbornene copolymer.

Alternatively, or in combination, the transparent surface may comprise or consist of a transparent ceramic material.

The term "transparent ceramic material" as used according to the present invention refers to a ceramic material which allows light, in particular having a wavelength of 650 nm to 1200 nm, in particular 650 nm to 1100 nm or 750 nm to 1200 nm, to pass through the ceramic material, in particular without appreciable scattering of the light.

Preferably, the transparent ceramic material is selected from the group consisting of silica (SiO₂), alumina (Al₂O₃), zirconia (ZrO₂) and combinations of at least two of the afore-mentioned transparent ceramic materials.

Principally, the thermal nanoparticles may be bonded to the surface of the instrument body by different ways. For example, the thermal nanoparticles may be bonded via a covalent bond, in particular using a functional molecule, or via an electrostatic interaction, or via a complexing reaction. A combination of these different ways to bond the thermal nanoparticles on the surface of the instrument body may also be contemplated within the scope of the present invention. The functional molecule is expediently a bi-functional molecule or a molecule having at least two reactive endings.

Preferably, the thermal nanoparticles are firmly or non-releasable, in particular covalently, bonded to the surface of the instrument body.

Advantageously, the thermal nanoparticles are non-cytotoxic.

Further, the thermal nanoparticles may have a shape selected from the group consisting of a rod shape, a cylindrical shape, a triangular shape, a pyramidal shape, a cubic shape, a spherical shape, a star shape and a combination of at least two of the afore-mentioned shapes.

Preferably, the thermal nanoparticles have a rod shape.

Further, the thermal nanoparticles, to be more precise the shape of the thermal nanoparticles, in particular the rod shape of the thermal nanoparticles, may have an aspect ratio from 1 to 10, in particular 2 to 7, preferably 3 to 5.

Advantageously, in case of the rod shape, the intensity of the LSR of the thermal nanoparticles is particularly high and the position may be easily tunable by the aspect ratio of the rod shape.

In a further embodiment of the invention, the thermal nanoparticles comprise or consist of a material selected from the group consisting of gold, silver, copper, zinc, titanium, a semiconductor, an oxide, a metal oxide, a non-metallic material such as silicone, or combinations of at least two of the afore-mentioned materials. Preferably, the material is gold. In other words, the thermal nanoparticles are preferably thermal nanoparticles comprising or consisting of gold.

Further, the thermal nanoparticles may be bonded to the surface of the instrument body in a density from 10 thermal nanoparticles/µm² to 1000 thermal nanoparticles/µm². The density of the thermal nanoparticles may depend on the shape and/or geometry and/or cross-section of the thermal nanoparticles.

Further, the thermal nanoparticles may be coated, in particular with a polymer. Preferably, the thermal nanoparticles may comprise a polymer coating and the surface of the instrument body in which the thermal nanoparticles are entrapped is a polymeric surface, wherein the polymer of the coating and the polymer of the polymeric surface are identical or different, preferably identical. If the polymer of the coating and the polymer of the polymeric surface are identical, a homogenous distribution of the thermal nanoparticles in the surface of the instrument body may be advantageously fostered. With respect to useful polymers, reference is made to the previously disclosed polymers in terms of the surface of the instrument body to which the thermal nanoparticles are bonded to and/or in which the thermal nanoparticles are entrapped.

Further, any light source which is capable of generating the optical wavelength within the desirable wavelength spectrum of the thermal nanoparticles can be used to irradiate the medical coagulation instrument. For example, a fluorescent or halogen lamp, a laser, an intense pulsed light source, a light-emitting diode, an incandescent or chemiluminescence light source or combinations of at least two of the afore-mentioned light sources may be used as a light source within the scope of the present invention.

The medical coagulation instrument may comprise any type of instrument body which is able to bond the thermal nanoparticles to which light can be coupled. For that purpose, the instrument body may be previously activated using any surface modification method known in the art. Only as mere exemplary, physic-chemical methods such as a treatment with active gases and vapours or irradiation (plasma), deposition of polymers from active gases and vapours (chemical vapour deposition), active gas or accelerated ion treatments (gas phase oxidation with ozone and/or ion beam), cross-linking of surface molecules, mechanical methods such as roughening, chemical methods such as physical absorption, chemical conjugation to surface groups, chemical modification of the surface, graft polymerization with radiation initiation or chemical initiation, coating of the surface of the instrument body with an active component or coating matrix which contains active components can be used.

The instrument body and/or thermal nanoparticles may also contain functionalized groups or may be functionalized by an activation process, with reactive groups. The reactive groups may be selected from the group consisting of fluoride, chloride, bromide, iodide, carbaldehyde, keto, carboxylate, cyano, nitro, amide, hydroxyl, amine, sulfate, sulfide, phosphate, phosphite, oxy, mercapto and thio. Further useful groups may be selected from the group consisting of complex forming groups, groups able to form hydrogen bonds, molecules containing ionic groups for ionic adsorption and a combination thereof.

Further, the instrument body and/or thermal nanoparticles may be functionalized with cross-linkers like imidoester cross-linker dimethyl suberimidate, N-hydroxysuccinimide-ester, formaldehyde, glutaraldehyde, or the like. Other useful bi-functional cross-linkers may be a BOC-amino containing compound, ethane thiol, mercapto-1-butanol, or the like.

Further, the instrument body and/or thermal nanoparticles may be functionalized with a spacer such as a silane, in particular aminopropyl triethoxy silane (APTES), in particular after having generated a hydroxyl-enriched surface, in particular hydroxyl-enriched metallic surface, of the instrument body and/or thermal nanoparticles, in particular by treating the instrument body and/or thermal nanoparticles with a piranha solution (H₂SO₄/H₂O₂), in particular for approximately 30 minutes, and by subsequent rinsing with deionized water.

Further, the surface of the thermal nanoparticles can be also modified in order to bond to the surface of the instrument body as described herein. This modification can be performed by using hetero- and/or homo-functional molecules being able to bind on one side to the surface of the thermal nanoparticles and on the other side to the instrument body, for example the modification of a surface of thermal nanoparticles comprising or consisting of gold with thiol containing reagents which has on the other side the desired functional group able to bind to the instrument body, particularly either covalently or by ionic interaction. These hetero- and/or homo-functional molecules preferably include all HS-R-functional groups, where -R relates to any alkyl or polyethylene glycol chain and the functional groups relate to any chemical group able to be activated and coupled to the instrument body's surface. Especially HS-R-COOH, HS-R-NH₂, HS-R-SH, HS-R-SO₃H and HS-R-N(CH₂)₃⁺ are suitable for the purpose of bonding the thermal nanoparticles to the surface of the instrument body.

Further, the thermal nanoparticles' surface can be modified by a polymer such as polyvinylpyrrolidone.

Alternatively, the instrument body may not require to be activated, neither may require the use of a functional molecule. In such a case, the instrument body can, by way of example, be made of a polymer or a copolymer which has active groups, such as free amino groups, in and/or on its surface. These active groups can directly bond thermal nanoparticles without the need to previously prepare the instrument body. Preferably, the bonding method is not reversible.

Further, the instrument body and/or thermal nanoparticles may be activated by cold plasma polymerization, for example activated by the deposition of molecules like PFM (pentafluorophenyl methacrylate) and/or ethylene diamine.

Further, the instrument body and/or thermal nanoparticles may be functionalized with a functional molecule, the functional molecule preferably being a diamine derivative.

With respect to further features and advantages of the thermal nanoparticles supporting localized surface plasmon resonance and methods for bonding the thermal nanoparticles on the surface of a suitable substrate, reference is made to the WO 2015/049267 A1.

Further, the medical coagulation instrument may be in the form of a sterile medical coagulation instrument, in particular steam sterilized or ethylene oxide sterilized medical coagulation instrument.

According to a second aspect, the invention refers to a method for manufacturing a medical coagulation instrument, in particular according to the first aspect. The method comprises the following steps:
- providing an instrument body or part of an instrument body for anchoring thermal nanoparticles supporting optical resonance, in particular localized surface plasmon resonance (LSPR),
- selecting thermal nanoparticles supporting optical resonance, in particular localized surface plasmon resonance, and
- bonding the selected thermal nanoparticles to a surface of the instrument body or to the part of an instrument body, preferably thereby forming a thermal coating,
wherein the thermal nanoparticles are capable of increasing their temperature by light irradiation in a wavelength range that matches with the wavelength of the optical resonance, in particular localized surface plasmon resonance, of the thermal nanoparticles.

The part of an instrument body may be in particular selected from the group consisting of a base or main portion, at least one distal tip portion, in particular two distal tip portions, proximal handling portion, common juncture and combinations of at least two of the afore-mentioned parts of the instrument body.

The provision of an instrument body or part of an instrument body for anchoring thermal nanoparticles supporting optical resonance, in particular localized surface plasmon resonance, may include one or more of the following treatments when it is required activate or functionalize the instrument body or part of an instrument body:
- activating the surface of the instrument body or part of an instrument body by a surface modification method;
- functionalising the surface of the instrument body or part of an instrument body with a functional molecule, which has at least two reactive endings; and/or
- functionalising the surface of the thermal nanoparticles with a functional molecule, which has at least two reactive endings.

It is understood by the description herein to the fact that there are instrument bodies or parts of an instrument body that do not require to be activated nor to be functionalized.

The abovementioned surface modification method may be selected from the group consisting of a treatment with active gases and vapours or irradiation; deposition of polymers from active gases and vapours; active gas or accelerated ion treatments; crosslinking of surface molecules; mechanical methods; chemical methods; graft polymerization with radiation initiation or chemical initiation; coating of the surface with an active component and coating matrix which contains active component.

More preferably, the surface modification method is a cold plasma polymerization.

Further, the functionalising of the surface of the instrument body or part of an instrument body and/or functionalising of the surface of the thermal nanoparticles is carried out with a functional molecule selected from a crosslinker, complex forming groups, groups able to form hydrogen bonds and/or molecules containing ionic groups for ionic adsorption.

More preferably, the functionalising of the surface of the instrument body or part of an instrument body and/or functionalising of the surface of the thermal nanoparticles is carried out with a diamine derivative.

For further features and advantages of the method, reference is made in its entirety to the description of the first invention aspect. The features and advantages mentioned there, in particular in terms of the medical coagulation instrument, instrument body and thermal nanoparticles, applies, mutatis mutandis, to the method according to the second aspect of the invention.

According to a third aspect, the invention refers to a further method for manufacturing a medical coagulation instrument, in particular according to the first aspect. The method comprises the following steps:
- selecting thermal nanoparticles supporting optical resonance, in particular localized surface plasmon resonance (LSPR), and
- entrapping the selected thermal nanoparticles during manufacture of an instrument body or during manufacture of a part of an instrument body in a surface of the instrument body or part of the instrument body with the instrument body or part of the instrument body being adapted or configured to anchor the thermal nanoparticles supporting optical resonance, in particular localized surface plasmon resonance,
wherein the thermal nanoparticles are capable of increasing their temperature by light irradiation in a wavelength range that matches with the wavelength of the optical resonance, in particular localized surface plasmon resonance, of the thermal nanoparticles.

The thermal nanoparticles may be entrapped in the surface of the instrument body or part of an instrument body by means of moulding, in particular injection moulding, or a sintering process.

For further features and advantages of the method, reference is made in its entirety to the description of the first and/or second invention aspect. The features and advantages mentioned there, in particular in terms of the medical coagulation instrument, instrument body, part of an instrument body and thermal nanoparticles, applies, mutatis mutandis, to the method according to the second aspect of the invention.

According to a fourth aspect, the invention refers to a medical coagulation kit comprising a medical coagulation instrument according to the first aspect of the present invention and at least one further kit component.

In particular, the at least one further kit component is selected from the group consisting of instructions for use, light source, in particular infrared light source, and combinations of at least two of the afore-mentioned further kit components.

The light is preferably an infrared light source, more preferably a near infrared light source. More specifically, the light source may be adapted or configured to emit light having a wavelength from 750 nm to 1200 nm, in particular 780 nm to 900 nm, preferably 800 nm to 840 nm, more preferably 800 nm to 820 nm, in particular 805 nm to 815 nm, preferably 810 nm.

Further, the light source may be in particular selected from the group consisting of fluorescent lamp, halogen lamp, laser, intense pulsed light source, light-emitting diode, incandescent light source, chemiluminescence light source and combinations of at least two of the aforementioned light sources.

For further features and advantages of the medical coagulation kit, reference is made in its entirety to the previous description, in particular to the description of the first invention aspect. The features and advantages mentioned there, in particular in terms of the medical coagulation instrument, instrument body and thermal nanoparticles, applies, mutatis mutandis, to the medical coagulation kit according to the fourth aspect of the invention.

According to a fifth aspect, the present invention refers to a method for coagulation of blood, in particular for sealing blood vessels, in particular arteries and/or veins, comprising the step of
- treating, in particular grasping, a body site, in particular of a human or non-human mammal, in need thereof with a medical coagulation instrument comprising an instrument body and thermal nanoparticles, wherein the thermal nanoparticles support optical resonance, in particular localized surface plasmon resonance (LSPR), and are bonded to and/or entrapped in a surface of the instrument body, wherein the thermal nanoparticles are capable of increasing their temperature by light irradiation in a wavelength range that matches with the wavelength of the optical resonance, in particular localized surface plasmon resonance, of the thermal nanoparticles, wherein the surface of the instrument body to which the thermal nanoparticles are bonded to and/or in which the thermal nanoparticles are entrapped is irradiated with infrared light, in particular near infrared light, in particular having a wavelength of 750 nm to 1200 nm, in particular 780 nm to 900 nm, preferably 800 nm to 840 nm, more preferably 800 nm to 820 nm, in particular 805 nm to 815 nm, preferably 810 nm, during treating the body site in need thereof.

Preferably, the body site to be treated comprises or is a blood vessel, in particular an artery and/or a vein. Typically, the blood vessel, in particular artery and/or vein, to be treated is an injured, in particular bleeding, blood vessel, in particular artery and/or vein.

For further features and advantages of the method, reference is made in its entirety to the previous description. The features and advantages mentioned there, in particular in terms of the medical coagulation instrument, instrument body and thermal nanoparticles, applies, mutatis mutandis, to the method according to the fifth aspect of the invention.

Further features and advantages of the invention will become clear from the following description of preferred embodiments in form of figures, figure descriptions and examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### BRIEF DESCRIPTION OF THE FIGURES

In the following, embodiments of the invention are described in detail with reference to the figure. The figure schematically show:

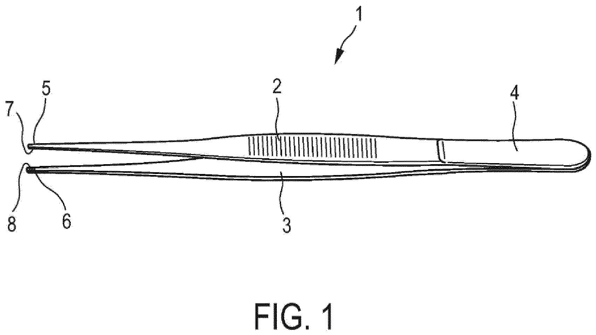

Fig. 1 an embodiment of a medical coagulation instrument according to the present invention.

### DETAILED DESCRIPTION OF THE FIGURES

According to Fig. 1, a medical instrument 1 according to the present invention comprises two instrument branches 2, 3, i.e., a first instrument branch 2 and a second instrument branch 3. The instrument branches 2, 3 are connected with each other by a common proximal juncture 4.

The instrument branches 2, 3 may be firmly connected with each other at the juncture 4. Alternatively, the instrument branches 2, 3 may be movably, in particular pivotally, interconnected at the juncture 4, in particular in the manner of a scissors (not shown).

Each of the two instrument branches 2, 3 has a distal tip portion 5, 6.

Thermal nanoparticles such as thermal gold nanoparticles are bonded to a surface of at least one of the two distal tip portions 5, 6, in particular at least to one of two opposing surfaces 7, 8 of the distal tip portions 5, 6.

In particular, the thermal nanoparticles may be bonded to both of the two opposing surfaces 7, 8 of the distal tip portions 5, 6.

Preferably, the thermal nanoparticles are in the form of a coating.

With respect to further features and advantages of the medical coagulation instrument as shown in Fig. 1, in particular in terms of the instrument body, instrument branches and the thermal nanoparticles, reference is made in its entirety to the previous description. The features and advantages described therein, in particular with respect to the medical coagulation instrument, instrument body, instrument branches, distal tip portions and thermal nanoparticles, do apply mutatis mutandis.

### EXAMPLE SECTION

### 1. Manufacture of thermal nanoparticles

### 1.1 Preparation of the seed:

Colloidal gold seeds were first prepared by mixing aqueous solutions of hexadecylcetyltrimethylammonium bromide (CTAB, 0.2 M, 5 mL) and hydrogen tetrachloroaurate(lll) hydrate (0.5 mM, 5 mL) both kept at 27°C. A freshly prepared aqueous solution of sodium borohydride (NaBH₄, 0.01 M, 0.6 mL) was then added, previously cooled to 4°C, under vigorous stirring for 2 minutes. At this point the seed was allowed to settle for 2hrs at 30"C to allow remaining NaBH₄ to evolve. This yielded a gold nanoparticle suspension of sizes between 1-2 nm, which were used as seed for the preparation of nanoparticles. This solution will be further referred as Seed Suspension.

### 1.2 Thermal-Nanoparticle's Growth:

The "growth solution" was now prepared and consisted of CTAB (0.2 M, 20 mL), to which varying amounts of silver nitrate stock (4 mM) was added depending on desired nanoparticle aspect ratio and allowed to mix under mild stirring. Hydrogen tetrachloroaurate(III) hydrate (1 mM, 20 mL) was added and gave rise to a yellow/brown solution. Once ascorbic acid (79 mM, 0.29 mL) was added to the yellow/brownish solution, the mixture should turn colorless. Next, 72 pL of aged Seed Suspension was added to the growth solution, mixed briefly and left undisturbed for 8 hrs at 30"C to prevent CTAB crystallization. Initial color change of the mixture should be noted after ca. 10 min. This procedure yielded a nanoparticle suspension presenting an LSPR maximum at around 820 nm (± 20nm) and a maximum absorption of 1.6 AU.

### 1.3 Preparation of-COOH Mix nanoparticles:

20 mL of the nanoparticles suspension obtained according to 1.2 were centrifuged twice at 14000 rpm, 30 minutes. Each time the supernatant was removed and replaced by a 4mM CTAB in water solution.

A solution mixture of the carboxylating reagents was prepared as follows: 97 mg of SH-PEG-COOH (Mw:3000) and 3 mg of mercapto undecyl carboxylic acid (MUA) were dissolved in 10 mL of water and the pH was adjusted to 7.

The nanoparticles suspension (20 mL) was then added of 2 mL of the mixture of carboxylating reagents (10 mg/ml) and placed in an ultrasound bath at 45 °C. The resulting mixture was sonicated during 30 minutes and then placed at 30 °C overnight.

The resulting carboxylated nanocomplex suspension was then centrifuged (14000 rpm, 30 minutes), the supernatant eliminated and finally redispersed with pure water to yield an absorption of around 3.5 AU at the SPR maximum.

### 2. Manufacture of a surgical tweezers modified with thermal gold nanoparticles

Thermal gold nanoparticles obtained according to 1.3 were bonded to the surface of the two distal tip portions (each made of polypropylene) of a surgical tweezers. The thermal gold nanoparticles were conjugated to the distal tip portions by 1-ethyl-3-[3-dimethylaminopropyl] carbodiimide hydrochloride (EDC) and N-hydroxysulfosuccinimide reactions (NHS). Briefly, 5 mg of NHS and 10 mg of ECD were dissolved in 1 mL of buffer 50 mM phosphate pH 7. Short after, 2 mL of GNR's solution were added to the buffer solution. After this step, the distal tip portions of the surgical tweezers were immersed in that solution for 4 hours. Right after, the distal tip portions were washed with pure water to remove any unbound thermal gold nanoparticles.

### 3. Manufacture of an arterial clamp modified with thermal gold nanoparticles

Thermal gold nanoparticles obtained according to 1.3 and suspended in cetyltrimethylammonium bromide (CTAB) were linked to an amino modified surface of two distal tip portions of an arterial clamp without further modification. The ability of metallic gold to complex with amino groups was exploited to form a great number of complex bonds leading to stable anchoring of gold nanoparticles to the distal tip portions. In practice, thermal gold nanoparticles suspended in CTAB after the growth step (100 mM CTAB) were centrifuged and the supernatant replaced by pure water to adjust the CTAB concentration to 3 mM and the nanoparticle concentration to an absorbance of 8 AU at 820 nm. The amino modified distal tip portions of the arterial clamp were immersed in this suspension and incubated overnight at 50 °C to perform the anchoring of thermal gold nanoparticles to the distal tip portions. The distal tip portions were then washed extensively with pure water.

### 4. Manufacture of a surgical clamp modified with thermal gold nanoparticles

Two polymeric distal tip portions of a surgical clamp, wherein each polymeric distal tip portion was made of polypropylene, were functionalized by carrying out a cold plasma polymerization to obtain reactive amino groups on the polymeric surface of the distal tip portions and citrate stabilized gold nanoparticles having a rod-shape were anchored to the surface of the two distal tip portions of the surgical clamp. For that purpose, the distal tip portions were incubated in a bath of rod-shaped gold nanoparticles in citrate buffer 20 mM pH 6,5. An excess of rod-shaped gold nanoparticles were used in order not to be limited by the quantity of rod-shaped gold nanoparticles. A determination of the content of gold nanoparticles was performed with ICP-OES (Inductively Coupled Plasma - Optical Emission Spectrometry) measurements.

## Claims

1. Medical coagulation instrument comprising an instrument body and thermal nanoparticles, wherein the thermal nanoparticles support optical resonance, in particular localized surface plasmon resonance, and are bonded to and/or entrapped in a surface of the instrument body, wherein the thermal nanoparticles are capable of increasing their temperature by light irradiation in a wavelength range that matches with the wavelength of the optical resonance, in particular localized surface plasmon resonance, of the thermal nanoparticles.

2. Medical coagulation instrument according to claim 1, **characterized in that** the surface of the instrument body to which the thermal nanoparticles are bonded to and/or in which the thermal nanoparticles are entrapped comprises a surface of a least one distal tip portion of the instrument body.

3. Medical coagulation instrument according to claim 1 or 2, **characterized in that** the instrument body comprises two distal tip portions, wherein the surface to which the thermal nanoparticles are bonded to and/or in which the thermal nanoparticles are entrapped comprises a surface of at least one of the two distal tip portions, preferably at least one surface of two opposing surfaces of the two distal tip portions.

4. Medical coagulation instrument according to claim 3, **characterized in that** one of the two distal tip portions is part of a first instrument branch of the instrument body and the other distal tip portion is part of a second instrument branch of the instrument body.

5. Medical coagulation instrument according to claim 4, **characterized in that** the first instrument branch and second instrument branch are pivotally coupled to each other.

6. Medical coagulation instrument according to claim 4, **characterized in that** the first instrument branch and second instrument branch are non-pivotally coupled to each other.

7. Medical coagulation instrument according to any of the claims 2 to 6, **characterized in that** the instrument body comprises a base portion to which the at least one distal tip portion, in particular the two distal tip portions are connected, in particular positively and/or force-fit connected and/or materially connected.

8. Medical coagulation instrument according to any of the preceding claims, **characterized in that** the medical coagulation instrument is in the form of a single use medical coagulation instrument and/or the at least one distal tip portion is in the form of at least one single use distal tip portion.

9. Medical coagulation instrument according to any of the preceding claims, **characterized in that** the medical coagulation instrument is in the form of a surgical coagulation instrument, in particular selected from the group consisting of surgical grasping instrument, surgical clamping instrument and surgical sealing instrument.

10. Medical coagulation instrument according to any of the preceding claims, **characterized in that** the medical coagulation instrument is selected from the group consisting of surgical tweezers, surgical scissors, surgical pliers, hemostatic clamps, arterial forceps, surgical staplers and scalpels.

11. Medical coagulation instrument according to any of the preceding claims, **characterized in that** the surface of the instrument body to which the thermal nanoparticles are bonded to and/or in which the thermal nanoparticles are entrapped is a metallic or polymeric surface.

12. Medical coagulation instrument according to any of the preceding claims, **characterized in that** the surface of the instrument body to which the thermal nanoparticles are bonded to and/or in which the thermal nanoparticles are entrapped is a transparent surface, in particular comprising a transparent polymer, preferably selected from the group consisting of polyethylene, polypropylene, polyimide, polydimethylsiloxane, polyethylene terephthalate, poly(methyl methacrylate), poly(phthalazinone ether sulfone ketone), poly(m-phenylene isophthalamide), polybenzoxazole, polycarbonate, cyclic olefin copolymer and combinations of at least two of the afore-mentioned transparent polymers, and/or a transparent ceramic material, preferably selected from the group consisting of silica (SiO₂), alumina (Al₂O₃), zirconia (ZrO₂) and combinations of at least two of the afore-mentioned transparent ceramic materials.

13. Medical coagulation instrument according to any of the preceding claims, **characterized in that** the thermal nanoparticles comprise or consist of a material selected from the group consisting of gold, silver, copper, zinc, titanium, a semiconductor, an oxide, a metal oxide, a non-metallic material such as silicone and combinations of at least two of the afore-mentioned materials.

14. Method for manufacturing a medical coagulation instrument, in particular according to any of the preceding claims, the method comprising the steps of:
- providing an instrument body for anchoring thermal nanoparticles supporting optical resonance, in particular localized surface plasmon resonance,
- selecting thermal nanoparticles supporting optical resonance, in particular localized surface plasmon resonance, and
- bonding the selected thermal nanoparticles to a surface of the instrument body,
wherein the thermal nanoparticles are capable of increasing their temperature by light irradiation in a wavelength range that matches with the wavelength of the optical resonance, in particular localized surface plasmon resonance, of the thermal nanoparticles
or
- selecting thermal nanoparticles supporting optical resonance, in particular localized surface plasmon resonance, and
- entrapping the selected thermal nanoparticles during manufacture of an instrument body or a part of an instrument body in a surface of the instrument body or part of the instrument body with the instrument body or part of the instrument body being adapted or configured to anchor the thermal nanoparticles supporting optical resonance, in particular localized surface plasmon resonance,
wherein the thermal nanoparticles are capable of increasing their temperature by light irradiation in a wavelength range that matches with the wavelength of the optical resonance, in particular localized surface plasmon resonance, of the thermal nanoparticles.

15. Medical coagulation kit comprising a medical coagulation instrument according to any of the preceding claims and at least one further kit component, in particular selected from the group consisting of instructions for use, light source, in particular infrared light source, and combinations of at least two of the aforementioned further kit components.
